# EUROPÄISCHE PATENTSCHRIFT

(11) **EP 1 024 793 B1**
(45) Veröffentlichungstag und Bekanntmachung des Hinweises auf die Patenterteilung: **16.01.2002**
(21) Anmeldenummer: 98955434.0
(22) Anmeldetag: 12.10.1998
(51) Int. Cl.: A61K 9/44

(54) **OSMOTISCHES ARZNEIMITTELFREISETZUNGSSYSTEM**
OSMOTIC MEDICAMENT RELEASING SYSTEM
SYSTEME OSMOTIQUE DE LIBERATION DE MEDICAMENT

(30) Priorität: 25.10.1997 DE 19747261
(43) Veröffentlichungstag der Anmeldung: 09.08.2000
(73) Patentinhaber: BAYER AG, 51368 Leverkusen (DE)
(72) Erfinder: KETTELHOIT, Stefan, D-51375 Leverkusen (DE); KANIKANTI, Ranga-Rao, D-51381 Leverkusen (DE); BRENDEL, Erich, D-42657 Solingen (DE); WEISEMANN, Claus, D-51429 Bergisch Gladbach (DE); CHANTRAINE, Ernst, D-51061 Köln (DE); EISELE, Michael, D-51469 Bergisch Gladbach (DE); BOSCHE, Patrick, D-51519 Odenthal (DE)
(86) Internationale Anmeldenummer: EP9806454
(87) Internationale Veröffentlichungsnummer: WO9921535

(56) Entgegenhaltungen:
- EP-A- 0 277 092
- EP-A- 0 740 934
- WO-A-96/40080
- WO-A-97/39050

## Beschreibung

Die vorliegende Erfindung betrifft ein oral zu verabreichendes osmotisches Arzneimittelfreisetzungssystem, das aus einer Hülle und einem wirkstoffhaltigen Kern besteht, sowie ein Verfahren zu dessen Herstellung. Die Erfindung betrifft weiter ein osmotisches Arzneimittelfreisetzungssystem zur Anwendung als Arzneimittel bei Menschen oder Tieren sowie die Verwendung des osmotischen Arzneimittelfreisetzungssystems zur Herstellung eines Arzneimittels zur Behandlung und/oder Prävention von Erkrankungen bei Menschen und Tieren.

Osmotische Arzneimittelfreisetzungssysteme sind grundsätzlich im Stand der. Technik bekannt. Dabei wird im allgemeinen ein osmotischer Druck als Energiequelle ausgenutzt, einen Arzneimittelwirkstoff mit kontrollierter Geschwindigkeit an das umgebende Medium abzugeben. Daher nennt man solche Systeme auch osmotische Pumpen. Eine weitgehend vollständige Übersicht über die osmotischen Arzneimittelfreisetzungssysteme findet sich in Journal of Controlled Release 35 (1995) 1-21. Danach unterscheidet man prinzipiell zwischen Mehrkammersystemen und Einkammersystemen. Das Einkammersystem besteht in seiner einfachsten Form aus einer konventionellen Tablette, die aus der Hülle einer semipermeablen Membran mit einer Austrittsöffnung und einem Kern, in der der Wirkstoff in fester Form vorliegt, besteht. Nach der oralen Verabreichung dringt Wasser durch die semipermeable Membran in den Kern ein, der Wirkstoff löst sich auf und wird durch eine Austrittsöffnung abgegeben (US-Patent No. 3.845.770). Dieses Prinzip eignet sich jedoch nur für sehr gut wasserlösliche Wirkstoffe, da nur diese einen ausreichend hohen osmotischen Druck erzeugen können. Speziell für schwerlösliche Wirkstoffe wurden daher sogenannte Doppelkammersysteme ("Push-Pull"-Systeme) entwickelt (US-Patent Nr. 4.111.202, Europäische Patentanmeldung Nr. 52 917). Die Herstellung solcher Zweikammersysteme ist jedoch technisch sehr aufwendig. Das Einkammersystem besitzt daher einen prinzipiellen Vorteil gegenüber den Mehrkammersystemen. Um die Vorteile des Einkammersystems bei schwerlöslichen Arzneimitteln dennoch zu nutzen, wurden zur Erzielung ausreichend hoher osmotischer Drücke im Innern der Tablette Einkammersysteme vorgeschlagen, deren Kern aus dem Wirkstoff und bestimmten polymeren Quellmitteln besteht, die beim Hinzutreten von Wasser durch die äußere semipermeable Membran aufquellen und zusammen mit dem darin teilweise suspendierten Wirkstoff aus der Öffnung freigesetzt werden. Der Auswahl bestimmter polymerer Quellmittel kommt bei diesem System eine entscheidende Bedeutung zu, da wie in der EP-A-0 277 092 bereits beschrieben, bestimmte Quellmittel wie z.B. Polyvinylpyrrolidon, Polyethylenoxid oder Polymethacrylat einen so hohen Quelldruck erzeugen, daß es nach kurzer Zeit zu einer vollständigen Aufsprengung der semipermeablen Hüllmembran kommt und der Wirkstoff in kurzer Zeit freigesetzt wird, anstatt, wie gewünscht, verzögert bzw. kontrolliert freigesetzt zu werden. Die EP-A-0 277 092 trifft zur Lösung dieses Problems daher eine bestimmte Auswahl hydrophiler polymerer Quelimittel, nämlich eine Mischung aus einem Vinylpyrrolidon-Vinylacetat-Copolymer und einem Ethylenoxidhomopolymer.

Die WO 96/40080 beansprucht in generischer Form osmotische Einkammersysteme, die einen Kern aus einem pharmazeutischen Wirkstoff, einem wasserlöslichen osmotischen Mittel und einem wasserquellbaren Polymer umfassen. Wie jedoch bereits in der EP-A-0 277 092 dargelegt wird, sind nicht alle polymeren hydrophilen Quellmittel für diese Einkammersysteme geeignet, und eine sorgfältige Auswahl muß getroffen werden, um die gewünschte kontrollierte Freisetzung des Wirkstoffes aus dem Einkammersystem zu gewährleisten. In den konkreten Ausführungsformen der WO 96/40080 werden als wasserquellbare Polymere u.a. Polyethylenoxid und Cellulose bzw. deren Derivate verwendet.

Ein osmotisches Arzneimittelfreisetzungssystem mit kontrollierter, d.h. im allgemeinen mit verzögerter Freisetzung, das aus einem Einkammersystem besteht, sollte grundsätzlich eine möglichst vollständige Freisetzung des Wirkstoffes ermöglichen, ohne daß es während der Freisetzung zu einem Aufreißen der Austrittsöffnung und somit zu unkontrollierter Wirkstofffreisetzung kommt. Bei den Einkammersystemen besteht jedoch häufig das Problem, daß ein nicht unerheblicher Anteil des Wirkstoffs in der Tablette verbleibt, da der im Innern der Tablette erzeugte osmotische Druck nicht ausreicht, den Wirkstoff vollständig freizusetzen. So besitzen die oben beschriebenen Systeme den Nachteil, daß sie den Wirkstoff nicht vollständig aus der Hüllmembran durch die Austrittsöffnung abgeben, so daß ein relativ hoher Anteil des Wirkstoffs nicht absorbiert wird und ungenutzt ausgeschieden wird. Wird jedoch auf der anderen Seite ein wasserquellbares Polymer verwendet, das einen sehr hohen osmotischen Druck erzeugt, kann dies zum Aufreißen oder gar zur vollständigen Sprengung der Tablette führen, so daß eine verzögerte, kontrollierte Freisetzung nicht erreicht wird.

Häufig weisen die osmotischen Arzneimittelfreisetzungssysteme des Stands der Technik auch das Problem auf, daß die unbeschichteten Tablettenkerne eine ungenügende mechanische Festigkeit aufweisen, was die nachfolgende Lackierung erschwert.

Grundsätzlich sollte ein osmotisches Arzneimittelfreisetzungssystem leicht herstellbar sein, aus preiswerten und pharmakologisch gut verträglichen Stoffen zusammengesetzt sein und es ermöglichen, ein günstiges Freisetzungsprofil des Wirkstoffs zu erreichen

Es wurde nun überraschend gefunden, daß eine Kombination zweier bestimmter hydrophiler wasserquellbarer Polymere in bestimmten Gewichtsanteilen als Kernbestandteile besonders geeignet ist, die oben beschriebenen gewünschten Eigenschaften eines osmotischen Einkammer-Arzneimittelfreisetzungssystems, das einen pharmazeutischen Wirkstoff, insbesondere ein Dihydropyridin umfaßt, zu erreichen. Die Erfinder der vorliegenden Erfindung fanden, daß die Kombination aus dem Heteropolysaccharid Xanthan und einem Vinylpyrrolidon-Vinylacetat-Copolymer als wasserquellbare Polymere in bestimmten Gewichtsmengen zu einer weitgehend vollständigen Freisetzung eines Wirkstoffes aus der Hülle führt, ohne daß es dabei zu einem Aufreißen der Öffnung und unkontrollierter Wirkstofffreisetzung kommt.

Ohne an eine Theorie gebunden zu sein, wird angenommen, daß das Xanthan in Kombination mit dem Vinylpyrrolidon-Vinylacetat-Copolymer insbesondere deshalb sehr günstige Ereisetzungseigenschaften bewirkt, da es strukturviskose Lösungen bildet, deren Viskosität beim Fließen unter dem Einfluß zunehmender Schubspannung abnimmt. Dies erlaubt offenbar eine besonders gleichförmige Freisetzung des Wirkstoffs aus der Austrittsöffnung, ohne daß es zum Einreißen der Membran kommt, und dabei wird der Wirkstoff über einen relativ langen Zeitraum gleichförmig und weitgehend vollständig freigesetzt.

Xanthan als wasserquellbares Polymer besitzt darüberhinaus gegenüber den in der EP-A-0 277 092 und der WO 96/40080 verwendeten Polyethylenoxiden den Vorteil der leichteren Handhabbarkeit, da es nicht den sogenannten TOMS-Effekt (Herabsetzung des Reibungswiderstands) aufweist. Ein weiterer Vorteil der Verwendung des Xanthans gegenüber der Verwendung von Polyethylenoxiden als wasserquellbare Polymere besteht darin, daß Polyethylenoxide in der Regel nur mit organischen Lösungsmitteln feucht granuliert werden (s. z.B. Beispiele der EP-A-0 277 092), so daß die Herstellung unter Explosionsschutz erfolgen muß, oder es wird trocken tablettiert (WO 96/40080), so daß die bekannten Nachteile der Trockentablettierung, wie schlechte Fließfähigkeit der Mischung der Kernbestandteile, Staubentwicklung sowie eine geringere Härte des Tablettenkerns auftreten.

Die vorliegende Erfindung überkommt die oben beschriebenen Probleme des Stands der Technik mit der Bereitstellung eines osmotischen Arzneimittelfreisetzungssystem, das besteht aus:
- einer Hülle aus einem wasserdurchlässigen, für die Komponenten des Kerns undurchlässigen Material, die mindestens eine Öffnung aufweist, und

- einem Kern, enthaltend

- 15 bis 35 Gew.-% eines pharmazeutischen Wirkstoffs
- 20 bis 50 Gew.-% Xanthan
- 10 bis 30 Gew.-% eines Vinylpyrrolidon-Vinylacetat-Copolymers,
wobei gegebenenfalls die Differenz zu 100 Gew.-% durch mindestens einen Be-standteil gebildet wird, der aus der Gruppe ausgewählt wird, die aus weiteren hydrophilen quellbaren Polymeren, osmotisch aktiven Zusätzen und pharmazeutisch annehmbaren Zusatzstoffen besteht, die Gew.-%-Angaben auf das Gesamtgewicht der Kernbestandteile bezogen sind und die Summe der Kernbestandteile zu 100 % aufaddiert.

Die Hülle des osmotischen Arzneimittelfreisetzungssystems besteht aus einem wasserdurchlässigen, für die Komponenten des Kerns undurchlässigen Material. Solche Hüllmaterialien sind im Prinzip bekannt und beispielsweise beschrieben in der EP-A-0 277 092. Zur Herstellung der Hülle eignen sich z.B. die literaturbekannten polymeren Stoffe, die im Gastrointestinaltrakt nicht metabolisiert werden, d.h. unverändert ausgeschieden werden (s. US-Patente Nr. 3.916.899 und Nr. 3.977.404). Beispielsweise können acylierte Cellulosederivate (Celluloseester), die durch Acetylgruppen ein- bis dreifach oder durch Acetylgruppen ein- bis zweifach und einen weiteren von Acetyl verschiedenen Acylrest substituiert sind, verwendet werden, z.B. Celluloseacetat, Cellulosetriacetat, Celluloseacetatethylcarbamat, Celluloseacetatphthalat, Celluloseacetatmethylcarbamat. Celluloseacetatsuccinat, Celluloseacetatdimethylaminoacetat, Celluloseacetatethylcarbonat, Celluloseacetatchloracetat, Celluloseacetatethyloxalat, Celluloseacetatmethylsulfonats, Cellulose-acetatbutylsulfonat. Celluseacetatpropionat, Celluloseacetatdiethylaminoacetat, Celluloseacetatoctat, Celluloseacetatlaurat, Celluloseacetat-p-toluolsulfonat, Celluloseacetatbutyrat und andere Celluloseacetatderivate sowie Agaracetat und Amyloseacetat. Als semipermeables Membranmaterial eignen sich auch Ethylcellulose und polymere Epoxide, Copolymere aus Alkylenoxid und Alkylglycidylethern, Polyglykole und Polymilchsäurederivate und weitere Derivate davon. Ferner können auch Mischungen von an sich wasserunlöslichen Acrylaten (z.B. ein Copolymerisat von Acrylsäureethylester und Methacrylsäuremethylester) verwendet werden. Auf die Hülle kann bei Bedarf ein Lichtschutzlack aufgebracht werden. Geeignete Materialien für den Lichtschutzlack sind z.B. Polymere, wie Hydroxypropylcellulose, Hydroxypropylmethylcellulose. in Kombination mit geeigneten Weichmachern wie z.B. Polyethylenglykol und Pigmenten wie z.B. Titandioxid oder Eisenoxide.

Die Mengen und die verwendeten Bestandteile für die Herstellung der Hülle des osmotischen Arzneimittelfreisetzungssystems beeinflussen in bekannter Weise die Eintrittsgeschwindigkeit der gastrointestinalen Flüssigkeit. Grundsätzlich nimmt die Eintrittsgeschwindigkeit der gastrointestinalen Flüssigkeit mit zunehmender Lackmenge ab.

Die Hülle des osmotischen Arzneimittelfreisetzungssystems der vorliegenden Erfindung weist mindestens eine Öffnung bzw. Passage auf, durch die der Wirkstoff zusammen mit den weiteren Kernbestandteilen allmählich austritt. Die Öffnung wird durch Laserbohren, mechanisches Bohren oder z.B. Stanzen in die Hülle eingebracht. Es können ein oder mehrere Öffnungen in der Hülle vorhanden sein. Die Größe der Öffnung beträgt bevorzugt 0,2 bis 1,6 mm, besonders bevorzugt 0,4 bis 1,2 mm. Die Beschaffenheit und die Herstellverfahren der Öffnung sind an sich bekannt und beispielsweise beschrieben in den US-Patenten Nr. 4063064, 4088864 und 3916899 sowie in der EP-B-0277092.

Der Kern des osmotischen Arzneimittelfreisetzungssystems der vorliegenden Erfindung enthält, bzw. besteht im wesentlichen aus den folgenden Bestandteilen:
- 15 bis 35 Gew.-% eines pharmazeutischen Wirkstoffs
- 20 bis 50 Gew.-% Xanthan
- 10 bis 30 Gew.-% eines Vinylpyrrolidon-Vinylacetat-Copolymers,
wobei gegebenenfalls die Differenz zu 100 Gew.-% durch mindestens einen Bestandteil gebildet wird, der aus der Gruppe ausgewählt wird, die aus weiteren hydrophilen quelibaren Polymeren, osmotisch aktiven Zusätzen und pharmazeutisch annehmbaren Zusatzstoffen besteht, die Gew.-%-Angaben auf das Gesamtgewicht der Kernbestandteile bezogen sind und die Summe der Kernbestandteile zu 100% aufaddiert.

Bevorzugt besteht der Kern aus:
- 20 bis 30 Gew.-% eines pharmazeutischen Wirkstoffs
- 25 bis 40 Gew.-% Xanthan
- 10 bis 20 Gew.-% eines Vinylpyrrolidon-Vinylacetat-Copolymers,
wobei gegebenenfalls die Differenz zu 100 Gew.-% durch mindestens einen Bestandteil gebildet wird, der aus der Gruppe ausgewählt wird, die aus weiteren hydrophilen quellbaren Polymeren, osmotisch aktiven Zusätzen und pharmazeutisch annehmbaren Zusatzstoffen besteht, die Gew.-%-Angaben auf das Gesamtgewicht der Kernbestandteile bezogen sind und die Summe der Kernbestandteile zu 100% aufaddiert.

Weitere auf dem Gebiet der osmotischen Arzneimittelfreisetzungssysteme übliche Bestandteile können enthalten sein, solange ihre Anwesenheit, die Lösung der eingangs beschriebenen Aufgabenstellung nicht beeinträchtigt.

Bei den im Kern befindlichen pharmazeutischen Wirkstoffen handelt es sich vorzugsweise um schwerlösliche Wirkstoffe mit einer maximalen Löslichkeit von ≤ 1 g in 1000 g Wasser, vor allem um solche, die auch noch im Dickdarm resorbiert werden, insbesondere um einen Wirkstoff der an sich bekannten Klasse der Dihydropyridine, wie sie zum Beispiel in der EP-A-0071819 beschrieben sind, z.B. Nifedipin und Nisoldipin. Sie wirken als Calciumantagonisten. Diese werden sowohl als Herzkreislaufmittel in der Indikation Bluthochdruck als auch in der Behandlung und Prävention ischämischer Gehirnerkrankungen eingesetzt.

Besonders bevorzugt wird Nifedipin verwendet.

Der Wirkstoff liegt im Kern des osmotischen Arzneimittelfreisetzungssystem der vorliegenden Erfindung in einer Menge von 15 bis 35 Gew.-%, bevorzugt 20 bis 30 Gew.-%, besonders bevorzugt 19 bis 23 Gew.-%, bezogen auf die Gesamtmenge der Kernbestandteile vor.

Das osmotische Arzneimittelfreisetzungssystem enthält als einen der wesentlichen Bestandteil des Kerns das hydrophile wasserquellbare Polymer Xanthan. Dabei handelt es sich um ein anionisches Heteropolysaccharid, das im Handel beispielsweise unter der Bezeichnung Rhodigel® (hergestellt durch Meyhall) erhältlich ist.

In einer bevorzugten Ausführungsform weist das Xanthan eine Partikelgröße von weniger als 800 µm auf. Eine Partikelgröße von mehr als 800 µm führt in einigen Fällen zu einem verschlechterten Freisetzungsverhalten. In einer besonders bevorzugten Ausführungsform beträgt die Partikelgröße des Xanthans weniger als 500 µm.

Das Xanthan liegt in einer Menge 20 bis 50 Gew.-%, bevorzugt 25 bis 40 Gew.-% besonders bevorzugt 28 bis 32 Gew.-%, bezogen auf die Gesamtmenge der Kernbestandteile vor.

Ein weiterer wesentlicher Bestandteil des Kerns des Arzneimittelfreisetzungssystems der vorliegenden Erfindung ist das Vinylpyrrolidon-Vinylacetat-Copolymer. Dieses Copolymer ist an sich bekannt und kann mit beliebigen Mischungsverhältnissen der Monomere hergestellt werden. Das bevorzugt verwendete kommerziell erhältliche Kollidon® VA64 (hergestellt durch BASF) ist z.B. ein 60:40- Copolymerisat. Es weist im allgemeinen einen Gewichtsmittelwert des Molekulargewichts Mw, bestimmt durch Lichtstreuungsmessungen, von etwa 45.000 bis etwa 70.000 auf. Die Menge des Vinylpyrrolidon-Vinylacetat-Copolymers im Kern des Arzneimittelfreisetzungssystems der vorliegenden Erfindung beträgt 10 bis 30 Gew.-%, bevorzugt 10 bis 20 Gew.-%, besonders bevorzugt 15 bis 20 Gew-%, bezogen auf das Gesamtgewicht der Kernbestandteile. Daraus ergibt sich ein bevorzugtes Gewichtsverhältnis von Xanthan zum Vinylpyrrolidon-Vinylacetat-Copolymer von 5 : 1 bis 2 : 3.

Bevorzugt enthält das osmotische Arzneimittelfreisetzungssystem der vorliegenden Erfindung lediglich Xanthan und das Vinylpyrrolidon-Vinylacetat-Copolymer als wasserquellbare Polymere als Kernbestandteile.

In einer besonders bevorzugten Ausführungsform der Erfindung enthält das osmotische Arzneimittelfreisetzungssystem mindestens einen osmotisch aktiven Zusatz und/oder mindestens einen pharmazeutisch annehmbaren Zusatzstoff.

Dabei ist ein osmotisches Arzneimittelfreisetzungssystem bevorzugt, dessen Kern enthält:
- 20 bis 30 Gew.-% eines pharmazeutischen Wirkstoffs
- 25 bis 40 Gew.-% Xanthan
- 10 bis 20 Gew.-% eines Vinylpyrrolidon-Vinylacetat-Copolymers,
- 10 bis 30 Gew.-% einer osmotisch aktiven Substanz,
- 8 bis 20 Gew.-% mindestens eines pharmazeutisch annehmbaren Zusatzstoffes,
wobei die Gew.-%-Angaben auf das Gesamtgewicht der Kernbestandteile bezogen sind und die Summe der Kernbestandteile zu 100% aufaddiert.

Obwohl es bevorzugt ist, daß das osmotische Arzneimittelfreisetzungssystem der vorliegenden Erfindung lediglich Xanthan und das Vinylpyrrolidon-Vinylacetat-Copolymer als wasserquellbare Polymere als Kernbestandteile enthält, können bei Bedarf weitere zusätzliche hydrophile quellbare Polymere im Kern enthalten sein, die z.B. ausgewählt werden aus Hydroxypropylcellulose, Hydroxypropyl-methylcellulose. Natriumcarboxymethylcellulose, Polyacrylsäuren bzw. deren Salze.

Die gegebenenfalls im Kern vorhandenen weiteren hydrophilen quellbaren Polymere liegen im Arzneimittelfreisetzungssystem der vorliegenden Erfindung in einer Menge vor, bei der die Lösung der eingangs beschriebenen Aufgabenstellung nicht beeinträchtigt ist.

Diese hydrophilen wasserquellbaren Polymere, die in der vorliegenden Erfindung verwendet werden, umfassen jedoch kein Polyethylenoxid (Polyethylenglykol), d.h. der Kern der Arzneimittelfreizusammensetzung der vorliegenden Erfindung ist frei von Polyethylenoxidzusätzen.

Die wahlweise im Kern des Arzneimittelfreisetzungssystems der vorliegenden Erfindung enthaltenen osmotisch aktiven Zusätze sind im Prinzip nicht beschränkt, und alle wasserlöslichen Stoffe, deren Verwendung in der Pharmazie unbedenklich ist, wie z.B. die in Pharmakopöen oder in "Hager" und "Remington Pharmaceutical Science" erwähnten wasserlöslichen Hilfsstoffe können verwendet werden. Insbesondere können wasserlösliche Salze von anorganischen oder organischen Säuren oder nichtionische organische Stoffe mit großer Wasserlöslichkeit wie z.B. Kohlehydrate, insbesondere Zucker, oder Aminosäuren verwendet werden. Zum Beispiel können die osmotisch aktiven Zusätze ausgewählt werden aus anorganischen Salzen wie Chloriden, Sulfaten, Carbonaten und Bicarbonaten von Alkali- oder Erdalkalimetallen, wie Lithium, Natrium, Kalium, Magnesium, Calcium sowie Phosphate, Hydrogen- oder Dihydrogenphosphate, Acetate, Succinate, Benzoate, Citrate oder Ascorbate davon. Des weiteren können Pentosen, wie Arabinose. Ribose oder Xylose, Hexosen, wie Glucose, Fructose, Galactose oder Mannose, Disaccharide wie Sucrose, Maltose oder Lactose oder Trisaccharide wie Raffinose verwendet werden. Zu den wasserlöslichen Aminosäuren zählen Glycin, Leucin, Alanin oder Methionin. Besonders bevorzugt wird Natriumchlorid verwendet. Diese osmotisch aktiven Zusätze sind bevorzugt in einer Menge von 10 bis 30 Gew.-%, besonders bevorzugt 15 bis 20 Gew.-%, bezogen auf die Gesamtmenge der kernbildenden Bestandteile enthalten.

Desweiteren kann der Kern des osmotischen Arzneimittelfreisetzungssystems der vorliegenden Erfindung einen oder mehrere pharmazeutisch annehmbare Zusatzstoffe enthalten, die ausgewählt werden aus: Pufferstoffen, wie z.B. Natriumbicarbonat, Sprengmitteln, wie z.B. Natriumcarboxymethylstärke, Gleitmitteln, wie z.B. Magnesiumstearat, Tablettierhilfsmitteln, Schutzkoiloiden, wie z.B. in der EP-B-0277092, S. 5, Z. 10-25 beschrieben, Weichmachern, wie z.B. in der EP-B-0277092, S. 5, Z. 29-32 beschrieben, Tensiden, wie z.B. in der EP-B-0277092, S. 5, Z. 33-44 beschrieben, Trägermaterialien, wie z.B. in der EP-B-0277092, S. 5, Z. 45-47 beschrieben.

In einer ganz besonders bevorzugten Ausführungsform der vorliegenden Erfindung umfaßt der Kern:
- 19 bis 23 Gew.-% eines pharmazeutischen Wirkstoffs
- 28 bis 32 Gew.-% Xanthan
- 15 bis 20 Gew.-% eines Vinylpyrrolidon-Vinylacetat-Copolymers,
- 15 bis 20 Gew.-% Natriumchlorid
- 5 bis 7 Gew.-% Natriumbicarbonat
- 6 bis 9 Gew.-% Natriumcarboxymethylstärke
- <1 Gew.-% Magnesiumstearat
wobei die Gew.-%-Angaben auf das Gesamtgewicht der Kernbestandteile bezogen sind und die Summe der Kernbestandteile zu 100% aufaddiert.

Das osmotische Arzneimittelfreisetzungssystems der vorliegenden Erfindung kann in verschiedenen Formen wie z.B. in der EP-B-0277092, S. 6, Z. 7-14 beschrieben vorliegen. Bevorzugt liegt es in Tablettenform vor.

Die Erfindung betrifft auch ein Verfahren zur Herstellung des erfindungsgemäßen osmotischen Arzneimittelfreisetzungssystems, bei dem die Bestandteile des Kerns miteinander vermischt werden, gegebenenfalls feucht oder trocken granuliert werden, tablettiert werden und der so entstandene Kern mit der Hülle beschichtet wird. Die Feuchtgranulation bewirkt häufig eine bessere Benetzbarkeit der Bestandteile des Tablettenkerns, wodurch die eintretende Gastrointestinalflüssigkeit den Kern besser durchdringt, was vielfach zu einer rascheren und vollständigeren Freisetzung des Wirkstoffs führt, so daß die Feuchtgranulation bevorzugt ist.

Das erfindungsgemäße osmotische Arzneimittelfreisetzungssystem wird zur Behandlung und/oder Prävention von Erkrankungen von Menschen und Tieren, wie z.B. bei Kreislauferkrankungen, Infektionen, Entzündungen, Schmerzzuständen, Asthma, Cancer, Malaria, Thrombosen, Diabetes, Herzrhythmusstörungen, Hypoglycaemien, Mycosen, Depressionen, Störungen des Salz- und Flüssigkeitshaushaltes, Stoffwechselstörungen wie z.B. Störungen des Fettstoffwechsels, koronaren Herzerkrankungen, Bluthochdruck, cerebralen Leistungsstörungen und zur Therapie neurologischer Defizite insbesondere nach Subarachnoidalblutung verwendet. Besonders bevorzugt werden die osmotischen Arzneimittelfreisetzungssysteme der vorliegenden Erfindung zur Behandlung von Bluthochdruck und koronaren Herzerkrankungen verwendet.

### Beispiel 1 und 2 (Tabletten mit trocken granulierten Bestandteilen)

Zusammensetzung

| Kern | | |
|---|---|---|
| | 1 | 2 |
| Nifedipin | 36,00 mg | 36,00 mg |
| Xanthan (Rhodigel® , Handelsprodukt. Meyhall) | 50,96 mg | 50,96 mg |
| Copolyvidon (Kollidon® VA64, Handelsprodukt, BASF, Vinylpyrrolidon-Vinylacetat-Copolymer) | 29,45 mg | 29,45 mg |
| Natriumchlorid | 28,71 mg | 28,71 mg |
| Natriumbicarbonat | 10,15 mg | 10,15 mg |
| Natriumcarboxymethylstärke | 12,74 mg | 12,74 mg |
| Aerosil | 0,85 mg | 0,85 mg |
| Mg-stearat | 0,68 mg | 0.68 mg |

| Hülle (osmotische Membran) | | |
|---|---|---|
| Celluloseacetat | 8,45 mg | 11,40 mg |
| Polyethylenglykol 3350 | 0,45 mg | 0,60 mg |
| | | |
| Tbl.-gewicht ca. | 178,5 mg | 181,6 mg |
| Tbl-format | 6r9 | 6r9 |

### Herstellungsverfahren

Nifedipin, Kollidon® VA64 (70 Gew.% der o.a. Menge), Rhodigel® Natriumchlorid und Natriumbicarbonat wurden gemischt und anschließend trocken granuliert. Das Granulat wurde mit Natriumcarboxymethylstärke, dem anteiligen Rest von Kollidon® VA64, Aerosil und Magnesiumstearat nachgemischt. Die Mischung wurde anschließend tablettiert. Die Tablettenkerne wurden mit einem die Bestandteile der osmotischen Membran enthaltenden organischen Lack beschichtet. Die beschichteten Tabletten wurden anschließend getrocknet. Die enstandenen Tabletten besaßen einen Durchmesser von 6 mm.

Anschließend wurde eine Öffnung von ca. 800 µm im Durchmesser bei jeder Tablette mit einem Handbohrer angebracht.

### Beispiel 3 und 4 (Tabletten mit feucht granulierten Bestandteilen)

Zusammensetzung

| Kern | | |
|---|---|---|
| | 3 | 4 |
| Nifedipin | 36,00 mg | 36,00 mg |
| Xanthan (Rhodigel® , Handelsprodukt, Meyhall) | 50,96 mg | 50,96 mg |
| Copolyvidon (Kollidon® VA64, Handelsprodukt, BASF, Vinylpyrrolidon-Vinylacetat-Copolymer) | 29,45 mg | 29,45 mg |
| Natriumchlorid | 28,71 mg | 28,71 mg |
| Natriumbicarbonat | 10,15 mg | 10,15 mg |
| Natriumcarboxymethylstärke | 12,74 mg | 12,74 mg |
| Aerosil | 0,90 mg | 0,90 mg |
| Mg-stearat | 0,50 mg | 0,50 mg |

| Hülle (osmotische Membran) | | |
|---|---|---|
| Celluloseacetat | 7,50 mg | 9,40 mg |
| Polyethylenglykol 3350 | 0,40 mg | 0,50 mg |
| | | |
| Tbl.-gewicht ca. | 177 mg | 179 mg |
| Tbl-format | 6r9 | 7r10 |

### Herstellungsverfahren

Rhodigel® , Natriumchlorid, Natriumbicarbonat und Natriumcarboxymethylstärke wurden gemischt und anschließend mit einer Suspension von Nifedipin und Kollidon® VA64 in Wasser feucht granuliert. Das Granulat wurde mit Aerosil und Magnesiumstearat nachgemischt. Die Mischung wurde anschließend tablettiert. Die Tablettenkerne wurden mit einem die Bestandteile der osmotischen Membran enthaltenden organischen Lack beschichtet. Die beschichteten Tabletten wurden anschließend getrocknet. Die enstandenen Tabletten besaßen einen Durchmesser von 6 mm bzw. 7 mm.

Anschließend wurden zwei Öffnungen von je ca. 600µm im Durchmesser bei jeder Tablette mit einem Handbohrer angebracht.

### Vergleichsbeispiel 1 (entsprechend Beispiel 3 der EP-A-0277092):

Zusammensetzung

| Kern | |
|---|---|
| Nifedipin | 50,00 mg |
| Polyox coagulant | 20,00 mg |
| Copolyvidon (Kollidon® VA64, Handelsprodukt, BASF, Vinylpyrrolidon-Vinylacetat-Copolymer) | 18,00 mg |
| Natriumchlorid | 20,00 mg |
| Mg-stearat | 2,00 mg |

| Hülle (osmotische Membran) | |
|---|---|
| Celluloseacetat | 11,20 mg |
| Polyethylenglykol 4000 | 1,50 mg |
| | |
| Tbl.-gewicht ca. | 122,7 mg |
| Tbl-format | 7r10 |

### Herstellungsverfahren

Nifedipin, Polyox coagulant, Kollidon® VA64, Natriumchlorid und Magnesiumstearat wurden gemischt. Die Mischung wurde anschließend jedoch zur Vermeidung der Verwendung organischer Lösungsmittel ohne vorherige Granulation direkt tablettiert. Die Tablettenkerne wurden mit einem die Bestandteile der osmotischen Membran enthaltenden organischen Lack beschichtet. Es wurde dabei Celluloseacetat Typ 398-10 anstatt Celluloseacetat Typ 320S eingesetzt, um die Verwendung von chlorierten Kohlenwasserstoffen zu vermeiden. Die beschichteten Tabletten wurden anschließend getrocknet. Die enstandenen Tabletten besaßen einen Durchmesser von 7 mm.

Anschließend wurde eine Öffnung von ca. 800 µm im Durchmesser bei jeder Tablette mit einem Handbohrer angebracht.

### Vergleichsbeispiel 2 (entsprechend Beispiel 1 der WO-96/40080):

Zusammensetzung

| Kern | |
|---|---|
| Nifedipin | 33,00 mg |
| Polyox WSR 303 | 27,50 mg |
| Polyox WSR N80 | 55,00 mg |
| Natriumcarboxymethylstärke | 82,50 mg |
| Lactose | 74.25 mg |
| Mg-stearat | 2,75 mg |

| Hülle (osmotische Membran) | |
|---|---|
| Celluloseacetat | 12,48 mg |
| Polyethylenglykol 400 | 0,78 mg |
| Saccharose micr. | 1,56 mg |
| Triacetin | 0,78 mg |
| Tbl.-gewicht ca. | 291 mg |
| | |
| Tbl-format | 9r15 |

### Herstellungsverfahren

Nifedipin, Polyox WSR 303, Polyox WSR N80, Natriumcarboxymethylstärke, Lactose und Magnesiumstearat wurden gemischt. Die Mischung wurde anschließend tablettiert. Die entstandenen Tablettenkerne waren sehr weich und ließen. sich nur schlecht weiterverarbeiten. Die Tablettenkerne wurden mit einem die Bestandteile der osmotischen Membran enthaltenden organischen Lack beschichtet. Die beschichteten Tabletten wurden anschließend getrocknet. Die enstandenen Tabletten besaßen einen Durchmesser von 9 mm. Anschließend wurde eine Öffnung von ca. 800 µm im Durchmesser bei jeder Tablette mit einem Handbohrer angebracht. Die in Figur 1 der WO-96/40080 gezeigten Freisetzungsmengen des Beispiels 1 der WO-96/40080 wurden unter den unten angegebenen Testbedingungen nicht gefunden.

Die in den Beispielen und Vergleichsbeispielen hergestellten osmotischen Arzneimittelfreisetzungssysteme wurden auf ihr Freisetzungsverhalten in der "Apparatur 2" der USP XXIII (The United States Pharmacopeia USP XXIII 1995, Seite 1791 bis 1792) gemäß der Rührflügelmethode untersucht. Dazu wurde die Wirkstofffreisetzung in einer gängigen Freisetzungsapparatur der Firma ERWEKA bestimmt. Die Tabletten wurden in Puffer pH=6,8 (10%ig) nach Deutschem Arzneibuch 9. Ausgabe mit Tensidzusatz bei 37°C und 100 Upm inkubiert. Innerhalb von 24 Stunden setzten die Tabletten den Wirkstoff gemäß Tabelle frei. Die Freisetzungsmengen in der Tabelle sind als prozentualer Anteil der freigesetzten Wirkstoffmenge, bezogen auf die gesamte ursprüngliche Wirkstoffmenge im Kern angegeben.

**Tabelle**

| Freisetzung von Wirkstoff aus Tabletten gemäß Beispiel 1-4 und aus Tabletten der Vergleichsbeispiele 1-2 | | | | | | |
|---|---|---|---|---|---|---|
| Freisetzung | Beispiel 1 | Beispiel 2 | Beispiel 3 | Beispiel 4 | Vergleichsbeispiel 1 | Vergleichsbeispiel 2 |
| 240 min. | 12,5% | 10% | 32,5% | 30,8% | 5% | 13,6% |
| 480 min. | 41,7% | 30% | 67,5% | 64,5% | 22% | 33,6% |
| 720 min. | 57,5% | 53,3% | 76,7% | 75,8% | 37% | 48,2% |
| 960 min. | 65% | 60,8% | 81,7% | 80,8% | 45% | 54,5% |
| 1440 min. | 70% | 71,7% | 87,5% | 86,7% | 51% | 62,7% |

Die Ergebnisse zeigen, daß das erfindungsgemäße osmotische Arzneimittelfreisetzungssystem den Wirkstoff im relevanten Zeitintervall - je nach angestrebter Freisetzungsrate - nahezu vollständig freisetzt, wohingegen die osmotischen Arzneimittelfreisetzungssyteme aus dem Stand der Technik den Wirkstoff am Ende der Freisetzung nur unvollständig freisetzen. Dabei ist davon auszugehen, daß nach einem Zeitraum von 24 Stunden eine Freisetzung an absorptionsrelevanten Stellen des Gastrointestinaltrakts nicht mehr stattfindet und die osmotischen Arzneimittelfreisetzungssysteme das Plateau ihrer Freisetzung erreicht haben. Die erfindungsgemäßen Beispiele 1 und 2 einerseits und 3 und 4 andererseits verdeutlichen den Einfluß der Feuchteranulation im Unterschied zur Trockengranulation. Wie oben dargelegt, bewirkt die Feuchtgranulation häufig eine bessere Benetzbarkeit der Bestandteile des Tablettenkerns, wodurch die eintretende Gastrointestinalflüssigkeit den Kern besser durchdringt, was zu einer rascheren und vollständigeren Freisetzung des Wirkstoffs führt. Die Feuchtgranulation ist daher bevorzugt. Der Einsatz der Feuchtgranulation wird durch die Verwendung der speziellen wasserquellbaren Polymere in dem erfindungsgemäßen osmotischen Arzneimittelfreisetzungssystem, welche im Gegensatz zu den im Stand der Technik verwendeten Polyethylenoxiden keiner organischen Lösungsmittel bedürfen, praktisch erst ermöglicht. Der Vergleich zwischen den erfindungsgemäßen Beispielen 1 und 2 bzw. 3 und 4 zeigt, daß eine höhere Hüllackauftragsmenge am Anfang der Freisetzung zu einer gewissen Verzögerung (Lag-Zeit) führt, und die Freisetzungsrate aufgrund der geringeren Eintrittsgeschwindigkeit der Gastrointestinalflüssigkeit verlangsamt wird. Über einen längeren Zeitraum werden jedoch weitgehend unabhängig von der Lackauftragsmenge etwa gleich hohe Freisetzungsmengen in den erfindungsgemäßen Beispielen erzielt.

Der Vergleich zwischen Beispiel 2 der Erfindung und Vergleichsbeispiel 1 zeigt, daß das erfindungsgemäße osmotische Arzneimittelfreisetzungssystem bei etwa gleicher Lackauftragsmenge und Lackzusammensetzung sowie vergleichbarem Herstellungsverfahren (Trockengranulation bzw. Direkttablettierung) eine deutlich höhere Endfreisetzung bei annähernd gleichen Anfangsfreisetzungsraten aufweist. Dies bedeutet, daß das erfindungsgemäße osmotische Arzneimittelfreisetzungssystem auch zu einem späteren Zeitpunkt noch mit einer relativ hohen Freisetzungsrate den Wirkstoff freisetzt, wenn die Freisetzung des Wirkstoffs des osmotischen Arzneimittelfreisetzungssystems des Vergleichsbeipiels praktisch bereits zum Erliegen gekommen ist. Das bedeutet, daß ein großer Anteil des Wirkstoffs des Vergleichsbeispiels in der Tablette verbleibt und somit ungenutzt ausgeschieden wird. Auch bei Vergleichsbeispiel 2 wird eine niedrige Freisetzungsrate in einem späteren Zeitpunkt der Freisetzung beobachtet.

## Patentansprüche

1. Osmotisches Arzneimittelfreisetzungssystem, bestehend aus
- einer Hülle aus einem wasserdurchlässigen, für die Komponenten des Kerns undurchlässigen Material, die mindestens eine Öffnung aufweist, und
- einem Kern, enthaltend
- 15 bis 35 Gew.-% eines pharmazeutischen Wirkstoffs
- 20 bis 50 Gew.-% Xanthan 10 bis 30 Gew.-% eines Vinylpyrrolidon-Vinylacetat-Copolymers,
wobei gegebenenfalls die Differenz zu 100 Gew.-% durch mindestens einen Bestandteil gebildet wird, der aus der Gruppe ausgewählt wird, die aus weiteren hydrophilen quellbaren Polymeren, osmotisch aktiven Zusätzen und pharmazeutisch annehmbaren Zusatzstoffen besteht, die Gew.-%-Angaben auf das Gesamtgewicht der Kernbestandteile bezogen sind und die Summe der Kernbestandteile zu 100% aufaddiert.

2. Osmotisches Arzneimittelfreisetzungssystem nach Anspruch 1, das einen Kern umfaßt, der enthält:
- 20 bis 30 Gew.-% eines pharmazeutischen Wirkstoffs
- 25 bis 40 Gew -% Xanthan
- 10 bis 20 Gew.-% eines Vinylpyrrolidon-Vinylacetat-Copolymers,
wobei gegebenenfalls die Differenz zu 100 Gew.-% durch mindestens einen Bestandteil gebildet wird, der aus der Gruppe ausgewählt wird, die aus weiteren hydrophilen quellbaren Polymeren, osmotisch aktiven Zusätzen und pharmazeutisch annehmbaren Zusatzstoffen besteht, die Gew.-%-Angaben auf das Gesamtgewicht der Kernbestandteile bezogen sind und die Summe der Kernbestandteile zu 100% aufaddiert.

3. Osmotisches Arzneimittelfreisetzungssystem nach Anspruch 1 oder 2, **dadurch gekennzeichnet, daß** der Kern mindestens einen osmotisch aktiven Zusatz enthält.

4. Osmotisches Arzneimittelfreisetzungssystem nach irgendeinem der Ansprüche 1 bis 3, **dadurch gekennzeichnet, daß** der Kern mindestens einen osmotisch aktiven Zusatz sowie mindestens einen pharmazeutisch annehmbaren Zusatzstoff enthält.

5. Osmotisches Arzneimittelfreisetzungssystem nach irgendeinem der Ansprüche 1 bis 4, das einen Kern umfaßt, der enthält:
- 20 bis 30 Gew.-% eines pharmazeutischen Wirkstoffs
- 25 bis 40 Gew.-% Xanthan
- 10 bis 20 Gew.-% eines Vinylpyrrolidon-Vinylacetat-Copolymers,
- 10 bis 30 Gew.-% einer osmotisch aktiven Substanz,
- 8 bis 20 Gew.-% mindestens eines pharmazeutisch annehmbaren Zusatzstoffes,
wobei die Gew.-%-Angaben auf das Gesamtgewicht der Kernbestandteile bezogen sind und die Summe der Kernbestandteile zu 100% aufaddiert.

6. Osmotisches Arzneimittelfreisetzungssystem nach irgendeinem der Ansprüche 1 bis 5, **dadurch gekennzeichnet, daß** der Wirkstoff ein schwerlöslicher Wirkstoffist.

7. Osmotisches Arzneimittelfreisetzungssystem nach irgendeinem der Ansprüche 1 bis 6, **dadurch gekennzeichnet, daß** der Wirkstoff ein schwerlöslicher Wirkstoff ist, der auch noch im Dickdarm resobiert wird.

8. Osmotisches Arzneimittelfreisetzungssystem nach irgendeinem der Ansprüche 1 bis 7, **dadurch gekennzeichnet, daß** der Wirkstoff ein Dihydropyridin ist.

9. Osmotisches Arzneimittelfreisetzungssystem nach irgendeinem der Ansprüche 1 bis 8, **dadurch gekennzeichnet, daß** der Wirkstoff Nifedipin ist.

10. Osmotisches Arzneimittelfreisetzungssystem nach irgendeinem der Anspruche 1 bis 9, **dadurch gekennzeichnet, daß** der osmotisch aktive Zusatz Natriumchlorid ist.

11. Osmotisches Arzneimittelfreisetzungssystem nach irgendeinem der Ansprüche 1 bis 10, **dadurch gekennzeichnet, daß** der pharmazeutisch annehmbare Zusatzstoff ausgewählt wird aus pharmazeutisch annehmbaren Pufferstoffen, pharmazeutisch annehmbaren Gleitmitteln, pharmazeutisch annehmbaren Sprengmitteln sowie pharmazeutisch annehmbaren Tablettierhilfsmitteln.

12. Osmotisches Arzneimittelfreisetzungssystem nach Anspruch 11, **dadurch gekennzeichnet, daß** der pharmazeutisch annehmbare Pufferstoff Natriumbicarbonat, das pharmazeutisch annehmbare Gleitmittel Magnesiumstearat und das pharmazeutisch annehmbare Sprengmittel Natriumcarboxymethylstärke ist.

13. Osmotisches Arzneimittelfreisetzungssystem nach irgendeinem der Ansprüche 1 bis 12, das einen Kern umfaßt, der enthält:
- 19 bis 23 Gew.-% eines pharmazeutischen Wirkstoffs
- 28 bis 32 Gew.-% Xanthan
- 15 bis 20 Gew.-% eines Vinylpyrrolidon-Vinylacetat-Copolymers,
- 15 bis 20 Gew.-% Natriumchlorid
- 5 bis 7 Gew.-% Natriumbicarbonat
- 6 bis 9 Gew.-% Natriumcarboxymethylstärke
- <1 Gew.-% Magnesiumstearat
wobei die Gew.-%-Angaben auf das Gesamtgewicht der Kernbestandteile bezogen sind und die Summe der Kernbestandteile zu 100% aufaddiert.

14. Osmotisches Arzneimittelfreisetzungssystem nach irgendeinem der Ansprüche 1 bis 13, **dadurch gekennzeichnet, daß** es in Tablettenform vorliegt.

15. Verfahren zur Herstellung des osmotischen Arzneimittelfreisetzungssystems nach irgendeinem der Ansprüche 1 bis 14, **dadurch gekennzeichnet, daß** die Bestandteile des Kerns miteinander vermischt werden, gegebenenfalls trocken oder feucht granuliert werden, tablettiert werden und der so entstandene Kern mit der Hülle beschichtet wird.

16. Osmotisches Arzneimittelfreisetzungssystem nach irgendeinem der Ansprüche 1 bis 14 zur Verwendung als Arzneimittel bei Menschen oder Tieren.

17. Verwendung des osmotischen Arzneimittelfreisetzungssystems nach irgendeinem der Ansprüche 1 bis 14 zur Herstellung eines Arzneimittels zur Behandlung von Bluthochdruck, koronaren Herzerkrankungen, cerebralen Leistungsstörungen und zur Therapie neurologischer Defizite nach Subarachnoidalblutung.

18. Verwendung des osmotischen Arzneimittelfreisetzungssystems nach Anspruch 17 zur Herstellung eines Arzneimittels zur Behandlung von Bluthochdruck und koronaren Herzerkrankungen.

## Claims

1. Osmotic pharmaceutical release system, consisting of
- a shell of a water-permeable material which is impermeable to the components of the core, which shell has at least one orifice, and
- a core comprising
- 15 to 35% by weight of an active pharmaceutical ingredient
- 20 to 50% by weight of xanthan
- 10 to 30% by weight of a vinylpyrrolidone/vinyl acetate copolymer,
where the difference from 100% by weight is formed where appropriate by at least one ingredient which is selected from the group consisting of further hydrophilic swellable polymers, osmotically active additions and pharmaceutically acceptable additives, the % by weight data are based on the total weight of the core ingredients, and the total of the core ingredients adds up to 100%.

2. Osmotic pharmaceutical release system according to Claim 1, which comprises a core which contains:
- 20 to 30% by weight of an active pharmaceutical ingredient
- 25 to 40% by weight of xanthan
- 10 to 20% by weight of a vinylpyrrolidone/vinyl acetate copolymer,
where the difference from 100% by weight is formed where appropriate by at least one ingredient which is selected from the group consisting of further hydrophilic swellable polymers, osmotically active additions and pharmaceutically acceptable additives, the % by weight data are based on the total weight of the core ingredients, and the total of the core ingredients adds up to 100%.

3. Osmotic pharmaceutical release system according to Claim 1 or 2, **characterized in that** the core contains at least one osmotically active addition.

4. Osmotic pharmaceutical release system according to any one of Claims 1 to 3, **characterized in that** the core contains at least one osmotically active addition and at least one pharmaceutically acceptable additive.

5. Osmotic pharmaceutical release system according to any one of Claims 1 to 4, which comprises a core which contains:
- 20 to 30% by weight of an active pharmaceutical ingredient
- 25 to 40% by weight of xanthan
- 10 to 20% by weight of a vinylpyrrolidone/vinyl acetate copolymer
- 10 to 30% by weight of an osmotically active substance
- 8 to 20% by weight of at least one pharmaceutically acceptable additive,
where the % by weight data are based on the total weight of the core ingredients, and the total of the core ingredients adds up to 100%.

6. Osmotic pharmaceutical release system according to any one of Claims 1 to 5, **characterized in that** the active ingredient is an active ingredient of low solubility.

7. Osmotic pharmaceutical release system according to any one of Claims 1 to 6, **characterized in that** the active ingredient is an active ingredient of low solubility which also undergoes absorption in the colon.

8. Osmotic pharmaceutical release system according to any one of Claims 1 to 7, **characterized in that** the active ingredient is a dihydropyridine.

9. Osmotic pharmaceutical release system according to any one of Claims 1 to 8, **characterized in that** the active ingredient is nifedipine.

10. Osmotic pharmaceutical release system according to any one of Claims 1 to 9, **characterized in that** the osmotically active addition is sodium chloride.

11. Osmotic pharmaceutical release system according to any one of Claims 1 to 10, **characterized in that** the pharmaceutically acceptable additive is selected from pharmaceutically acceptable buffer substances, pharmaceutically acceptable lubricants, pharmaceutically acceptable disintegrants and pharmaceutically acceptable tabletting aids.

12. Osmotic pharmaceutical release system according to Claim 11, **characterized in that** the pharmaceutically acceptable buffer substance is sodium bicarbonate, the pharmaceutically acceptable lubricant is magnesium stearate and the pharmaceutically acceptable disintegrant is sodium carboxymethylstarch.

13. Osmotic pharmaceutical release system according to any one of Claims 1 to 12, which comprises a core which contains:
- 19 to 23% by weight of an active pharmaceutical ingredient
- 28 to 32% by weight of xanthan
- 15 to 20% by weight of a vinylpyrrolidone/vinyl acetate copolymer
- 15 to 20% by weight of sodium chloride
- 5 to 7% by weight of sodium bicarbonate
- 6 to 9% by weight of sodium carboxymethylstarch
- < 1% by weight of magnesium stearate
where the % by weight data are based on the total weight of the core ingredients, and the total of the core ingredients adds up to 100%.

14. Osmotic pharmaceutical release system according to any one of Claims 1 to 13, **characterized in that** it is in tablet form.

15. Process for the production of the osmotic pharmaceutical release system according to any one of Claims 1 to 14, **characterized in that** the ingredients of the core are mixed together, where appropriate are dry- or wet-granulated and are tabletted, and the core produced in this way is coated with the shell.

16. Osmotic pharmaceutical release system according to any one of Claims 1 to 14, for use as pharmaceutical for humans or animals.

17. Use of the osmotic pharmaceutical release system according to any one of Claims 1 to 14 for the production of a pharmaceutical for the treatment of high blood pressure, coronary heart disease, cerebral dysfunctions and for the therapy of neurological deficits after subarachnoid haemorrhage.

18. Use of the osmotic pharmaceutical release system according to Claim 17 for the production of a pharmaceutical for the treatment of high blood pressure and coronary heart disease.

## Revendications

1. Système osmotique de libération de médicament, constitué
- d'une enveloppe qui est perméable à l'eau et imperméable aux composants du noyau et qui présente au moins une ouverture, et
- d'un noyau contenant
- 15 à 35 en poids d'une substance pharmaceutique active
- 20 à 50 % en poids de xanthane
- 10 à 30 % en poids d'un copolymère vinylpyrrolidone-acétate de vinyle,
le complément éventuel à 100 % en poids étant formé par au moins un constituant qui est choisi dans le groupe comprenant d'autres polymères hydrophiles gonflants, des additifs à activité osmotique et des additifs acceptables du point de vue pharmaceutique, les indications de pourcentages en poids étant basées sur le poids total des constituants du noyau et la somme des constituants du noyau totalisant 100 %.

2. Système osmotique de libération de médicament suivant la revendication 1, qui comprend un noyau contenant :
- 20 à 30 % en poids d'une substance pharmaceutique active
- 25 à 40 % en poids de xanthane
- 10 à 20 % en poids d'un copolymère vinylpyrrolidone-acétate de vinyle,
le complément éventuel à 100 % en poids étant formé par au moins un constituant qui est choisi dans le groupe comprenant d'autres polymères hydrophiles gonflants, des additifs à activité osmotique et des additifs acceptables du point de vue pharmaceutique, les pourcentages en poids indiqués étant basés sur le poids total des constituants du noyau et la somme des constituants du noyau totalisant 100 %.

3. Système osmotique de libération de médicament suivant la revendication 1 ou 2, **caractérisé en ce que** le noyau contient au moins un additif à activité osmotique.

4. Système osmotique de libération de médicament suivant l'une quelconque des revendications 1 à 3, **caractérisé en ce que** le noyau contient au moins un additif à activité osmotique ainsi qu'au moins un additif acceptable du point de vue pharmaceutique.

5. Système osmotique de libération de médicament suivant l'une quelconque des revendications 1 à 4, qui comprend un noyau contenant :
- 20 à 30 % en poids d'une substance pharmaceutique active
- 25 à 40 % en poids de xanthane
- 10 à 20 % en poids d'un copolymère vinylpyrrolidone-acétate de vinyle,
- 10 à 30 % en poids d'une substance à activité osmotique,
- 8 à 20 % en poids d'au moins un additif acceptable du point de vue pharmaceutique,
les indications de pourcentages en poids étant basées sur le poids total des constituants du noyau et la somme des constituants du noyau totalisant 100 %.

6. Système osmotique de libération de médicament suivant l'une quelconque des revendications 1 à 5, **caractérisé en ce que** la substance active est une substance active difficilement soluble.

7. Système osmotique de libération de médicament suivant l'une quelconque des revendications 1 à 6, **caractérisé en ce que** la substance active est une substance active difficilement soluble qui est encore résorbée dans le côlon.

8. Système osmotique de libération de médicament suivant l'une quelconque des revendications 1 à 7, **caractérisé en ce que** la substance active est une dihydropyridine.

9. Système osmotique de libération de médicament suivant l'une quelconque des revendications 1 à 8, **caractérisé en ce que** la substance active est la nifédipine.

10. Système osmotique de libération de médicament suivant l'une quelconque des revendications 1 à 9, **caractérisé en ce que** l'additif à activité osmotique est le chlorure de sodium.

11. Système osmotique de libération de médicament suivant l'une quelconque des revendications 1 à 10, **caractérisé en ce que** l'additif acceptable du point de vue pharmaceutique est choisi entre des tampons acceptables du point de vue pharmaceutique, des agents de glissement acceptables du point de vue pharmaceutique, des agents de désintégration acceptables du point de vue pharmaceutique ainsi que des auxiliaires acceptables du point de vue pharmaceutique pour la confection de comprimés.

12. Système osmotique de libération de médicament suivant la revendication 11, **caractérisé en ce que** le tampon acceptable du point de vue pharmaceutique est le bicarbonate de sodium, l'agent de glissement acceptable du point de vue pharmaceutique est le stéarate de magnésium et l'agent de désintégration acceptable du point de vue pharmaceutique est le sel de sodium du carboxyméthylamidon.

13. Système osmotique de libération de médicament suivant l'une quelconque des revendications 1 à 12, qui comprend un noyau contenant :
- 19 à 23 % en poids d'une substance pharmaceutique active
- 28 à 32 % en poids de xanthane
- 15 à 20 % en poids d'un copolymère vinylpyrrolidone-acétate de vinyle,
- 15 à 20 % en poids de chlorure de sodium,
- 5 à 7 % en poids de bicarbonate de sodium,
- 6 à 9 % en poids de sel de sodium de carboxyméthylamidon,
- moins de 1 % en poids de stéarate de magnésium,
les indications de pourcentages en poids étant basées sur le poids total des constituants du noyau et la somme des constituants du noyau totalisant 100 %.

14. Système osmotique de libération de médicament suivant l'une quelconque des revendications 1 à 13, **caractérisé en ce qu'**il se présente sous la forme de comprimés.

15. Procédé de production du système osmotique de libération de médicament suivant l'une quelconque des revendications 1 à 14, **caractérisé en ce qu'**on mélange les constituants du noyau, on granule éventuellement le mélange à sec ou par voie humide, on le transforme en comprimés et on recouvre de l'enveloppe le noyau ainsi produit.

16. Système osmotique de libération de médicament suivant l'une quelconque des revendications 1 à 14, destiné à être utilisé comme médicament en médecine humaine ou en médecine vétérinaire.

17. Utilisation du système osmotique de libération de médicament suivant l'une quelconque des revendications 1 à 14 pour la production d'un médicament destiné au traitement de l'hypertension, de maladies coronariennes, de troubles des capacités cérébrales et pour la thérapie de déficits neurologiques après une hémorragie subarachnoïdienne.

18. Utilisation du système osmotique de libération de médicament suivant la revendication 17 pour la préparation d'un médicament destiné à traiter l'hypertension et des maladies coronariennes.
